# EUROPEAN PATENT SPECIFICATION

(11) **EP 2 909 173 B1**
(45) Date of publication and mention of the grant of the patent: **27.03.2019**
(21) Application number: 13783838.9
(22) Date of filing: 17.10.2013
(51) Int. Cl.: C07D 213/24, C09B 1/52

(54) **DIMERIC ANIONIC ANTHRAQUINONE DYES**
ANIONISCHE DIMERE ANTHRACHINONFARBSTOFFE
COLORANTS ANTHRAQUINONES ANIONIQUES DIMÈRES

(30) Priority: 20.10.2012 DE 102012021081
(43) Date of publication of application: 26.08.2015
(73) Proprietor: Archroma IP GmbH, 4153 Reinach (CH)
(72) Inventor: GISLER, Markus, 4310 Rheinfelden (CH)
(74) Representative: Ricker, Mathias
(86) International application number: PCT/EP2013/003120
(87) International publication number: WO 2014/060103

(56) References cited:
- WO-A1-2006/003120
- FR-A1- 2 197 937
- US-A- 2 913 465

## Description

This invention relates to novel dimeric anionic anthraquinone dyes, their methods of preparation and their use for dyeing organic substrates, such as natural or synthetic polyamides and polyurethanes. Dimeric anthraquinone dyes are known from documents US2913465 and FR2197937.

Methylene-bridged dimeric anionic anthraquinone dye structures of formula (II) 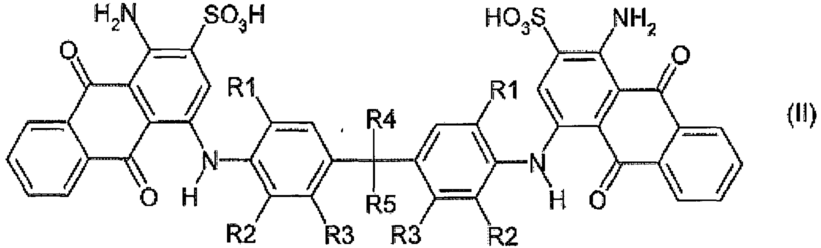 are derivatives of C.I. Acid Blue 127:1, C.I. 61133
(formula (II), R1, R2, R3, R4, R5 = H).

Compared with their partly very bright monomeric precursors of formula (III), 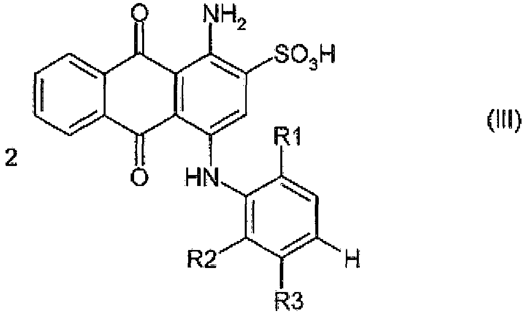 the doubled systems of formula (II) build up better on polyamide fibers, but their dyeings have a much duller and more greenish hue.

It was found that the novel dyes of the present invention, which conform to formula I (having a pyridyl radical attached to the bridging carbon atom and having one hydrogen atom) surprisingly still exhibit a clean and bright hue for the dyeing, as well as the unchanged good build-up on the fiber, despite the doubling.

The invention provides compounds of formula (I) 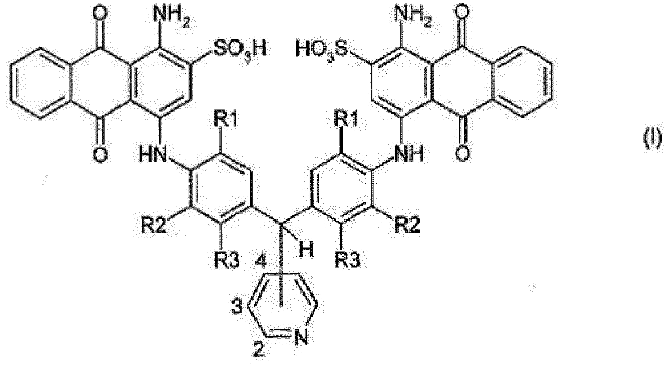 as free acid, as salt or as mixed salt, where
R1, R2 and R3 are each independently hydrogen, C₁₋₄alkyl or C₁₋₄alkyloxy.

The C₁₋₄alkyl and C₁₋₄alkoxy radicals mentioned are preferablyunsubstituted and unbranched.

R1 and R2 are each preferably methyl, ethyl and methoxy, more preferably methyl and methoxy.
R3 is preferably hydrogen or methyl, more preferably hydrogen.

In preferred compounds of formula (I), the pyridine ring attaches in the 4-position to the dye molecule.

In particularly preferred compounds of formula (I), the pyridine ring is substituted in the 4-position and
R1 and R2 are each methyl or methoxy, especially methyl, and
R3 is hydrogen.

The invention also provides a method of preparing compounds of formula (I). The compounds of formula (I) according to the present invention are obtainable under known conditions in
- an acid-catalyzed condensation reaction from their monomeric precursors of formula (III), and a pyridine carbaldehyde (reaction A1) 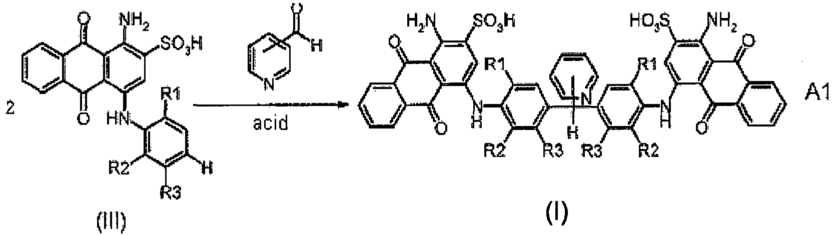
- or by means of a doubled copper-catalyzed Ullmann reaction from 1-amino-4-bromoanthraquinone-2-sulfonic acid (bromaminic acid, formula (IV), CAS No. [116-81-4] and 4,4'-diaminodiphenylmethane derivatives, of formula (V) (reaction B1). Syntheses of this type, carried out in aqueous alcohol, are described for example in the patent document US-2156887.

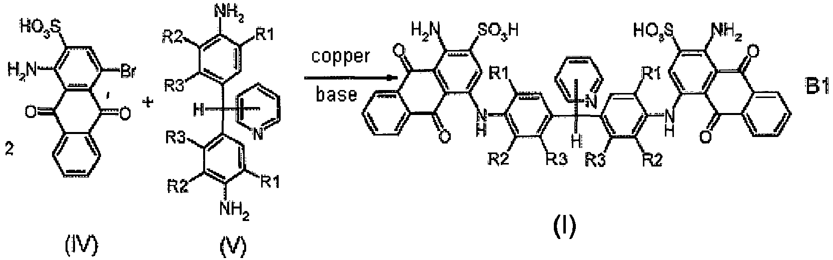

The acid-catalyzed reaction (reaction A1) of two molecules of formula (III) with one equivalent of a pyridinecarbaldehyde 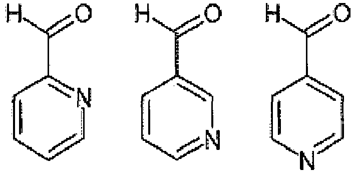 is the preferred method.

The reaction can be catalyzed using protic or Lewis acids, but it is preferable to use dilute sulfuric acid for this. It is particularly preferable to perform the reaction in 60 to 80 wt% sulfuric acid. The reaction can be carried out in a temperature range of 10 to 100°C, in which case the monomeric anthraquinone precursor of formula (III) is preferably suspended in the sulfuric acid at 10 to 30°C and the suspension is heated to 50-70°C after the pyridinecarbaldehyde has been added.

Depending on the reaction and/or isolation conditions, the dyes of formula (I) can be obtained as free acid, as salt or as mixed salt which contains for example one or more cations selected from alkali metal ions, for example sodium ion, or an ammonium ion or alkylammonium cation, for example mono-, di- or trimethyl- or -ethylammonium cations. The dye can be converted by conventional techniques from the free acid into a salt or into a mixed salt or vice versa or from one salt form into another. If desired, the dyes can be further purified by diafiltration, in which case unwanted salts and synthesis by-products are separated from the crude anionic dye.

The removal of unwanted salts and synthesis by-products and partial removal of water from the crude dye solution is carried out by means of a semipermeable membrane by applying a pressure whereby the dye is obtained without the unwanted salts and synthesis by-products as a solution and if necessary as a solid body in a conventional manner.

The dyes of formula (I) and their salts are particularly suitable for dyeing or printing fibrous material consisting of or containing natural or synthetic polyamides in bright blue shades. The dyes of formula (I) and their salts are suitable for producing inkjet printing inks and for using these inkjet printing inks to print fibrous material which consists of natural or synthetic polyamides or cellulose (paper, for example).

The invention accordingly provides from another aspect for the use of the dyes of formula (I), their salts and mixtures for dyeing and/or printing fibrous materials consisting of natural or synthetic polyamides. A further aspect is the manufacture of inkjet printing inks and their use for printing fibrous materials consisting of natural or synthetic polyamides.

Dyeing is carried out as per existing processes, for example the dyeing processes described in Ullmanns Encyklopädie der technischen Chemie, 4th edition, 1982, volume 22, pages 658-673 or in the book by M. Peter and H.K. Rouette, Grundlagen der Textilveredlung, 13th edition, 1989, pages 535-556 and 566-574. Preference is given to dyeing in the exhaust process at a temperature of 30 to 140°C, more preferably 80 to 120°C and most preferably at a temperature of 80 to 100°C, and at a liquor ratio of 3:1 to 40:1.

The substrate to be dyed can be present in the form of yarn, woven fabric, loop-formingly knitted fabric or carpet, for example. Fully-fashioned dyeings are even perfectly possible on delicate substrates, examples being lambswool, cashmere, alpaca and mohair. The dyes of the invention are particularly useful for dyeing fine-denier fibers (microfibers).

The dyes of the present invention and their salts are highly compatible with known acid dyes. Accordingly, the dyes of formula (I), their salts or mixtures can be used alone in a dyeing or printing process or else as a component in a combination shade dyeing or printing composition together with other acid dyes of the same class, i.e., with acid dyes possessing comparable dyeing properties, for example fastness properties and exhaustion rates from the dyebath onto the substrate. The dyes of the present invention can be used in particular together with certain other dyes having suitable chromophores: The dyeings of the dye according to the present invention can be shaded with suitable greenish blue dyes, for example C.I. Acid Blue 127:1, or green anionic dyes, for example C.I. Acid Green 40.

Combined with suitable anionic yellow dyes, for example C.I. Acid Yellow 236, or bright green dyes, for example C.I. Acid Green 81, bright green dyeings are achievable.

Bright bluish violet to violet hues are obtained in combination with red to violet dyes, for example C.I. Acid Violet 54 or C.I. Acid Violet 48.

The ratio in which the dyes are present in a combination shade dyeing or printing composition is dictated by the hue it is desired to obtain.

The novel dyes of formula (I), as stated above, are very useful for dyeing natural and synthetic polyamides, i.e., wool, silk and all nylon types, on each of which extremely bright dyeings having a high fastness level, especially good lightfastness and good wetfastnesses (washing, alkaline perspiration) are obtained. The dyes of formula (I) and their salts have a high rate of exhaustion. The ability of the dyes of formula (I) and their salts to build up is likewise very good. On-tone dyeings on the recited substrates are of outstanding quality. All dyeings also have a constant hue under artificial light. The fastness to decatizing and boiling is also good.

One decisive advantage of the novel dyes is that they are metal-free and deliver very level dyeings.

The compounds according to the invention can be used as an individual dye or else, owing to their good compatibility, as a combination element with other dyes of the same class having comparable dyeing properties, for example with respect to general fastnesses, exhaustion value. The combination shade dyeings obtained have similar fastnesses to dyeing with the individual dye.

In the examples which follow, parts and percentages are by weight and temperatures are reported in degrees Celsius.

### Preparation Example 1a

191 parts of 1-amino-4-bromoanthraquinone-2-sulfonic acid are suspended in a mixture of 800 parts of water and 400 parts of ethanol. 10 parts of 15% sodium carbonate solution are added to adjust the suspension to 9.5. 140 parts of 2,6-xylidine and also 2 parts of copper bronze are introduced into the reaction mixture. The reaction mixture is heated to 70°C and stirred at 70°C for 16 hours. Thereafter, the temperature of the heating bath is raised to 110-115°C and the alcohol is distilled off. The temperature of the heating bath is raised to 145-150°C.

The reaction mixture distills again. A total of 1350 parts of water are slowly added dropwise to the reaction mixture in the course of 4 h while the same amount of distillate is collected. Thereafter, the reaction mixture is filtered off. Drying at 60°C in vacuo and grinding gives 190 parts of a bluish violet powder which conforms to formula (1a), CAS Registry number [50408-06-05].

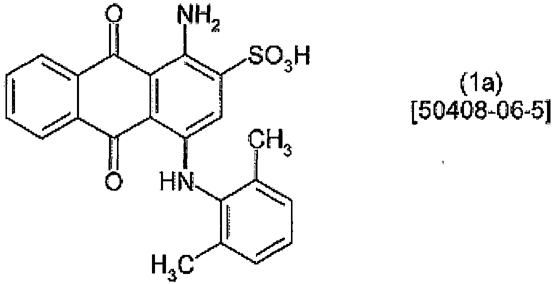

### Preparation Example 1b

190 parts of the dye powder obtained under Preparation Example 1a were introduced into 1240 parts of 80% sulfuric acid at room temperature, which is followed by stirring. Thereafter, 25.3 parts of pyridine-4-carbaldehyde, CAS Registry number [872-85-5], are added and the reaction mixture is heated to 60°C in the course of one hour. After about 90 minutes of stirring at 60°C, the reaction is substantially complete. The reaction mixture is pumped into 2700 parts of water at 65°C in the course of 30 minutes. The dark blue suspension is filtered off hot. Drying at 60°C in vacuo and grinding gives 230 parts of a bluish violet powder of formula (1b).

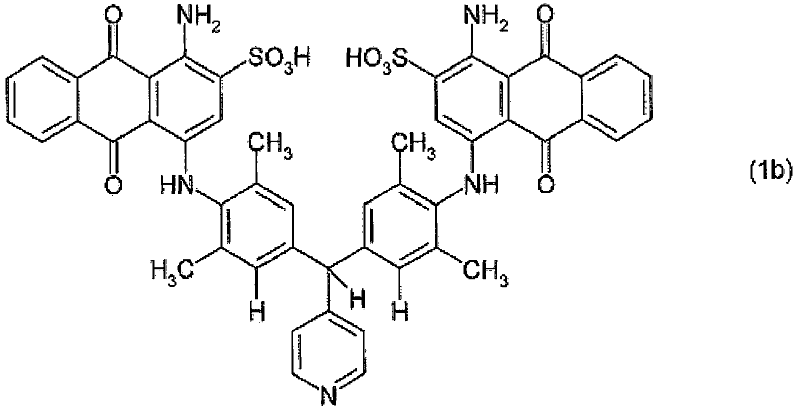

### Preparation Example 1c

The first half (95 parts) of the dye powder obtained under Preparation Example 1a are introduced into 1240 parts of 80% sulfuric acid at room temperature, before stirring for 1 h. Thereafter, 25.3 parts of pyridine-4-carbaldehyde, CAS Registry number [872-85-5], are added, which is followed by stirring for a further hour. After addition of the second half (95 parts) of the dye powder obtained under Example 1a, the reaction mixture is heated to 60°C in the course of one hour. After about 90 minutes of stirring at 60°C, the reaction is substantially complete. The reaction mixture is pumped into 2700 parts of water at 65°C in the course of 30 minutes. The dark blue suspension is filtered off hot. Drying at 60°C in vacuo and grinding gives 235 parts of a bluish violet powder. The dye likewise conforms to formula 1b.

The dye of formula 1b dyes polyamides or wool in extremely bright blue shades having good light- and wetfastnesses and good build-up. The wavelengths of maximum absorbance in the UV/VIS are 585 and 626 nm.

### Preparation Examples 2-3

The table which follows contains dyes prepared similarly to the method described in Preparation Example 1 by using the corresponding starting materials. These dyes provide blue dyeings on polyamide fibers and wool with very good light- and wetfastnesses. (Me is methyl, Et is ethyl, -OMe is methoxy.)

### Preparation Examples 2-9

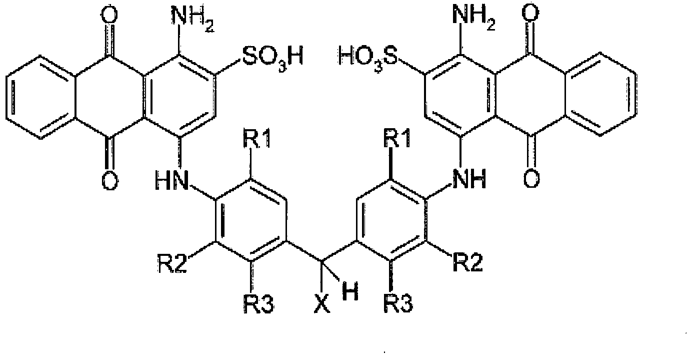

| Preparation Example | R1 | R2 | R3 | X | λₘₐₓ [nm] |
|---|---|---|---|---|---|
| 2 | Me | Me | H | 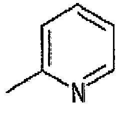 | 584/626 |
| 3 | Me | Me | H | 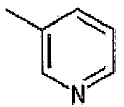 | 583/625 |
| 4 | H | H | H | 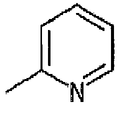 | 627 |
| 5 | H | H | H | 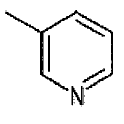 | 628 |
| 6 | H | H | H | 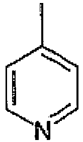 | 628 |
| 7 | Me | Et | H | 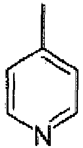 | 584/626 |
| 8 | OMe | Me | H | 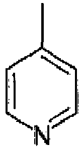 | 585/628 |
| 9 | OMe | H | Me | 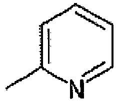 | 628 |

### Use Example A

A dyebath at 40°C, consisting of 2000 parts of water, 1 part of a weakly cation-active leveling agent which is based on an ethoxylated aminopropyl fatty acid amide and which has affinity for dye, 0.25 part of the dye of formula 1b from Preparation Examples 1b and 1c and adjusted to pH 5 with 1-2 parts of 40% aqueous acetic acid, is entered with 100 parts of woven nylon-6 fabric. After 10 minutes at 40°C the dyebath is heated to 98°C at a rate of 1°C per minute and then left at the boil for 45-60 minutes. Thereafter, it is cooled down to 70°C in the course of 15 minutes. The dyeing is removed from the bath, rinsed with hot and then with cold water and dried. The result obtained is a bright blue polyamide dyeing possessing good light- and wetfastnesses and showing good build-up.
Use Example A was repeated with the dyes of Preparation Examples 2-9 to similar effect with regard to levelness, light- and wetfastnesses and build-up.

### Use Example B

A dyebath at 40°C, consisting of 2000 parts of water, 1 part of a weakly cation-active leveling agent which is based on an ethoxylated aminopropyl fatty acid amide and which has affinity for dye, 0.3 part of the dye from Preparation Example 1 and adjusted to pH 5.5 with 1-2 parts of 40% aqueous acetic acid, is entered with 100 parts of woven nylon-6,6 fabric. After 10 minutes at 40°C the dyebath is heated to 120°C at a rate of 1.5°C per minute and then left at 120°C for 15-25 minutes. Thereafter, it is cooled down to 70°C in the course of 25 minutes. The dyeing is removed from the bath, rinsed with hot and then with cold water and dried. The result obtained is a bright blue polyamide dyeing possessing good light- and wetfastnesses and showing good build-up.
Use Example B was repeated with the dyes of Preparation Examples 2-9 to similar effect with regard to levelness, light- and wetfastnesses and build-up.

### Use Example C

A dyebath at 40°C, consisting of 4000 parts of water, 1 part of an amphoteric leveling agent which is based on a sulfated, ethoxylated fatty acid amide and has weak affinity for dye, 0.4 part of the dye of formula 1b from Preparation Example 1 and adjusted to pH 5 with 1-2 parts of 40% aqueous acetic acid, is entered with 100 parts of woven wool fabric. After 10 minutes at 40°C the dyebath is heated to boiling at a rate of 1°C per minute and then left at the boil for 40-60 minutes. Thereafter, it is cooled down to 70°C in the course of 20 minutes. The dyeing is removed from the bath, rinsed with hot and then with cold water and dried. The result obtained is a bright blue wool dyeing possessing good light- and wetfastnesses and showing good build-up.
Use Example C was repeated with the dyes of Preparation Examples 2-9 to similar effect with regard to levelness, light- and wetfastnesses and build-up.

### Use Example D

100 parts of woven nylon-6 material are padded with a 50°C liquor consisting of
- 40: parts of the dye of Preparation Example 1,
- 100: parts of urea,
- 20: parts of a nonionic solubilizer based on butyldiglycol,
- 15 - 20: parts of acetic acid (to adjust the pH to 4),
- 10: parts of a weakly cation-active leveling agent which is based on an ethoxylated aminopropyl fatty acid amide and has affinity for dye, and
- 810 - 815: parts of water (to make up to 1000 parts of padding liquor).

The material thus impregnated is rolled up and left to dwell in a steaming chamber under saturated steam conditions at 85-98°C for 3-6 hours for fixing. The dye is then rinsed with hot and cold water and dried. The result obtained is a bright blue polyamide dyeing having good levelness, good light- and wetfastnesses and good build-up.

Use Example D was repeated with the dyes of Preparation Examples 2-9 to similar effect with regard to levelness, light- and wetfastnesses and build-up.

### Use Example E

A textile cut pile sheet material composed of nylon-6 and having a synthetic base fabric is padded with liquor containing per 1000 parts
- 1: part of dye of Preparation Example 1,
- 4: parts of a commercially available thickener based on carob flour ether,
- 2: parts of a nonionic ethylene oxide adduct of a higher alkylphenol,
- 1: part of 60% aqueous acetic acid.
This is followed by printing with a paste which per 1000 parts contains the following components:
- 20: parts of commercially available alkoxylated fatty alkylamine (displace product),
- 20: parts of a commercially available thickener based on carob flour ether.

The print is fixed in saturated steam at 100°C for 6 minutes, rinsed and dried. The result obtained is a level-colored cover material with a blue and white pattern.
Use Example E was repeated with the dyes of Preparation Examples 2-9 to similar effect with regard to levelness, light- and wetfastnesses and build-up.

### Use Example F

3 parts of the dye from Preparation Examples 1-9 were each dissolved in 82 parts of demineralized water and 15 parts of diethylene glycol at 60°C. Cooling down to room temperature gives a blue printing ink which is very highly suitable for inkjet printing on paper, or polyamide and wool textiles.

## Claims

1. A compound of formula (I) 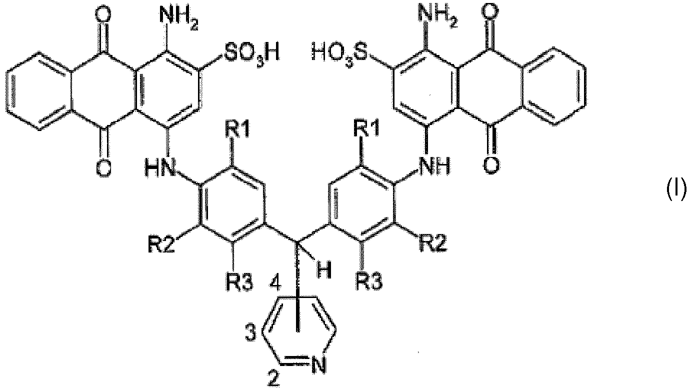 as free acid, as salt or as mixed salt,
where R1, R2 and R3 are each independently hydrogen, C₁₋₄ alkyl or C₁₋₄ alkyloxy.

2. The compound as claimed in claim 1 wherein the C₁₋₄ alkyl and C₁₋₄ alkoxy radicals are unsubstituted and unbranched.

3. The compound as claimed in claim 1 or 2 wherein R1 and R2 are each methyl, ethyl or methoxy.

4. The compound as claimed in one or more of claims 1 to 3 wherein R1 and R2 are each methyl or methoxy.

5. The compound as claimed in one or more of claims 1 to 4 wherein R3 is hydrogen or methyl.

6. The compound as claimed in one or more of claims 1 to 5 wherein R3 is hydrogen.

7. The compound as claimed in one or more of claims 1 to 6 wherein the pyridine ring attaches in the 4-position to the dye molecule.

8. The compound as claimed in one of more of claims 1 to 7 wherein the pyridine ring is substituted in the 4-position and R1 and R2 are each methyl or methoxy, and R3 is hydrogen.

9. The compound as claimed in claim 8 wherein R1 and R2 are each methyl.

10. A method of preparing a compound of formula (I) as claimed in one or more of claims 1 to 9, by an acid-catalyzed condensation reaction as per the following reaction scheme:

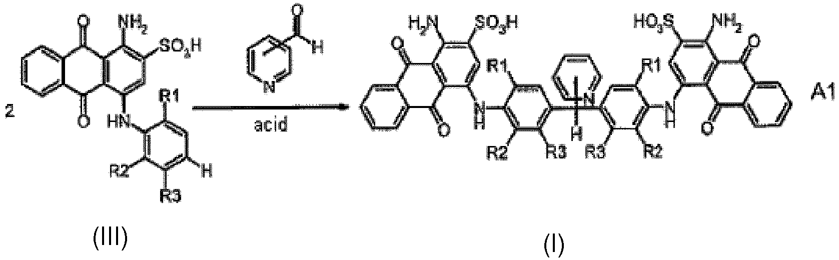

11. A method of preparing a compound of formula (I) as claimed in one or more of claims 1 to 9, as per the following reaction scheme:

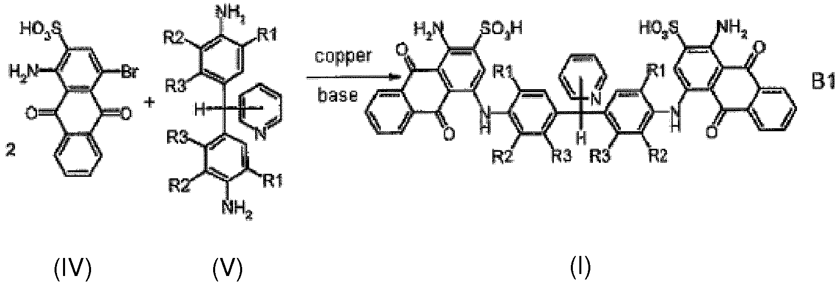

12. The use of compounds of formula (I) as claimed in one or more of claims 1 to 9 for dyeing or printing fibrous material consisting of or containing natural or synthetic polyamides.

13. The use as claimed in claim 12 for dyeing or printing wool, silk or nylon.

14. The use of compounds of formula (I) as claimed in one or more of claims 1 to 9 in the manufacture of inkjet inks.

## Patentansprüche

1. Verbindung der Formel (I) 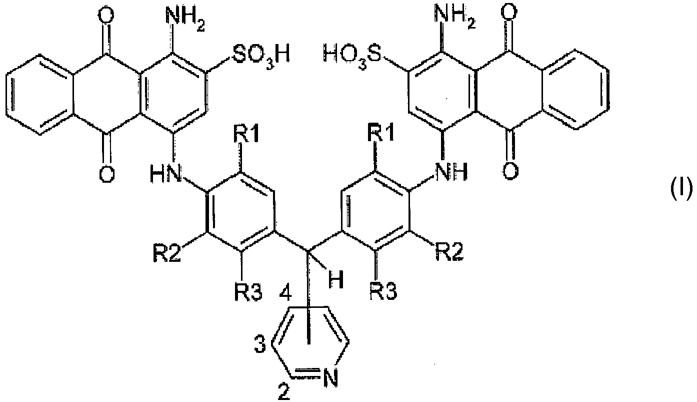 als freie Säure, als Salz oder als Mischsalz,
in der R1, R2 und R3 unabhängig voneinander Wasserstoff, C₁₋₄ Alkyl oder C₁₋₄ sind.

2. Verbindung, nach Anspruch 1, wobei die C₁₋₄ Alkyl und die C₁₋₄ Alkoxy Radikale unsubstituiert oder unverzweigt sind.

3. Verbindung nach Anspruch 1 oder 2, wobei R1 und R2 jeweils Methyl, Ethyl oder Methoxy sind.

4. Verbindung nach einem oder mehreren der Ansprüche 1 bis 3, wobei R1 und R2 jeweils Methyl oder Methoxy sind.

5. Verbindung nach einem oder mehreren der Ansprüche 1 bis 4, wobei R3 Wasserstoff oder Ethyl ist.

6. Verbindungen nach einem oder mehreren der Ansprüche 1 bis 5, wobei R3 Wasserstoff ist.

7. Verbindung nach einem oder mehreren der Ansprüche 1 bis 6, wobei der Pyridinring in der 4-Position an das Farbstoffmolekül anbindet.

8. Verbindung nach einem oder mehreren der Ansprüche 1 bis 7, wobei der Pyridyinring in der 4-Position substituiert ist und R1 und R2 jeweils Methyl oder Methoxy sind und R3 Wasserstoff ist.

9. Verbindung nach Anspruch 8, wobei R1 und R2 jeweils Methyl sind.

10. Verfahren zur Herstellung einer Verbindung nach Formel (I) nach einem oder mehreren der Ansprüche 1 bis 9, durch eine säurekatalysierte Kondensationsreaktion nach folgendem Reaktionsschema:

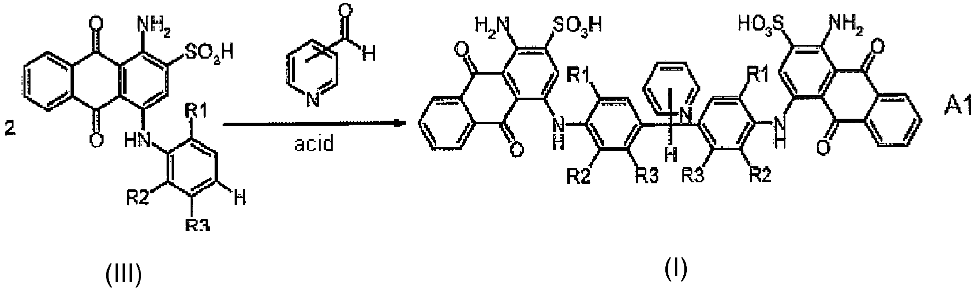

11. Verfahren zur Herstellung einer Verbindung nach Formel (I) nach einem oder mehreren der Ansprüche 1 bis 9, nach folgendem Reaktionsschema:

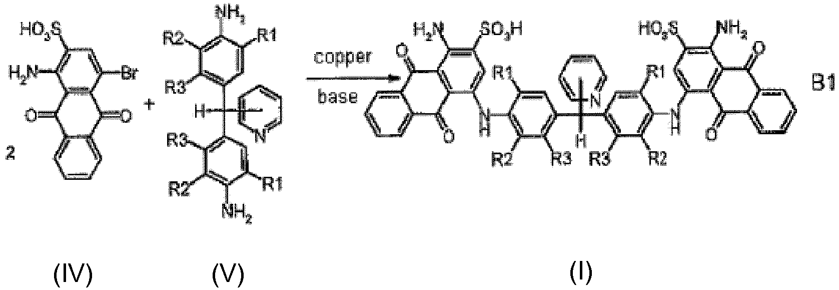

12. Verwendung von Verbindungen nach Formel (I) nach einem oder mehreren der Ansprüche 1 bis 9 zum Bedrucken von faserigem Material, die aus synthetischem Polyamid bestehen oder synthetisches Polyamid beinhalten.

13. Verwendung nach Anspruch 12 für das Färben oder das Bedrucken von Wolle, Seide oder Nylon.

14. Verwendung von Verbindungen nach Formel (I) nach einem oder mehreren der Ansprüche 1 bis 9 in der Herstellung von Tintenstrahltinte.

## Revendications

1. Composé de formule (I) 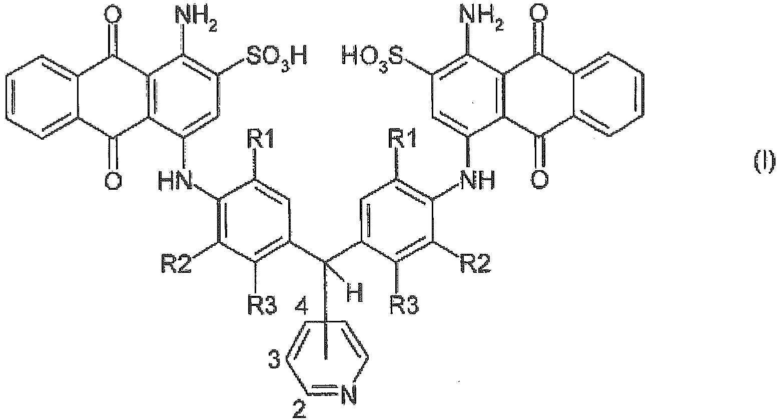 sous une forme d'acide, de sel, ou de mélange de sels,
dans lequel R1, R2 et R3 représentent chacun indépendamment un atome d'hydrogène, un radical alkyle en C₁ à C₄ ou alkyloxy en C₁ à C₄.

2. Composé selon la revendication 1 dans lequel les radicaux alkyle en C₁ à C₄ ou alkyloxy en C₁ à C₄ sont non substitués et non ramifiés.

3. Composé selon la revendication 1 ou 2 dans lequel R1 et R2 représentent chacun un radical méthyle, éthyle ou méthoxy.

4. Composé selon une ou plusieurs des revendications 1 à 3 dans lequel R1 et R2 représentent chacun un radical méthyle ou méthoxy.

5. Composé selon une ou plusieurs des revendications 1 à 4 dans lequel R3 représente un atome d'hydrogène ou un radical méthyle.

6. Composé selon une ou plusieurs des revendications 1 à 5 dans lequel R3 représente un atome d'hydrogène.

7. Composé selon une ou plusieurs des revendications 1 à 6 dans lequel le cycle pyridine se fixe en position 4 à la molécule de colorant.

8. Composé selon une ou plusieurs des revendications 1 à 7 dans lequel le cycle pyridine est substitué en position 4 et
R1 et R2 représentent chacun un radical méthyle ou méthoxy, et
R3 représente un atome d'hydrogène.

9. Composé selon la revendication 8 dans lequel R1 et R2 représentent chacun un radical méthyle.

10. Procédé de préparation d'un composé de formule (I) selon une ou plusieurs des revendications 1 à 9, par une réaction de condensation catalysée par un acide selon le schéma réactionnel suivant :

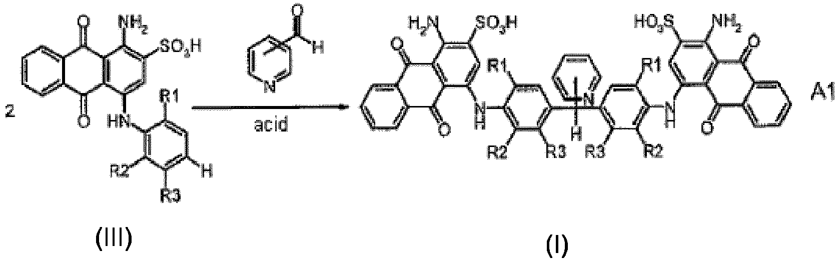

11. Procédé de préparation d'un composé de formule (I) selon une ou plusieurs des revendications 1 à 9, selon le schéma réactionnel suivant :

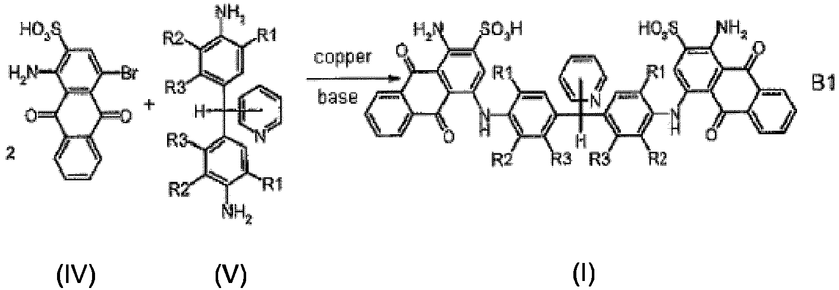

12. Utilisation des composés de formule (I) selon une ou plusieurs des revendications 1 à 9 pour colorer ou imprimer un matériau fibreux constitué de ou contenant des polyamides naturels ou synthétiques.

13. Utilisation selon la revendication 12 pour colorer ou imprimer de la laine, de la soie ou du nylon.

14. Utilisation des composés de formule (I) selon une ou plusieurs des revendications 1 à 9 dans la fabrication d'encres pour imprimante à jet d'encre.
